# EUROPEAN PATENT APPLICATION

(11) **EP 4 116 409 A1**
(43) Date of publication of application: **11.01.2023**
(21) Application number: 22182776.9
(22) Date of filing: 04.07.2022
(51) Int. Cl.: C12N 1/20, A23K 10/16, A23L 29/00, A23L 33/135, A23K 50/80, A23J 1/00

(54) **MICROBIAL BIOMASS-BASED FEED PRODUCTS**

(30) Priority: 06.07.2021 US 202117368601
(71) Applicant: Oakbio Inc., Sunnyvale, CA 94089 (US)
(72) Inventor: SEFTON, Brian A., Boise, ID, 83702 (US); COLEMAN, William J., Boise, ID, 83702 (US)
(74) Representative: Mollekopf, Gerd Willi

(57) **Abstract**

High protein and high nutritional products comprising a mix of specific bacterial species, and methods of producing the same, are provided. Products can be produced via a co-culture of axenic source strains grown on simple gas feedstocks, such as carbon dioxide, hydrogen and oxygen, as can be provided by industrial waste gases. A consortium of bacterial strains specially selected for this purpose is cultured in an aqueous culture medium.

## Description

### BACKGROUND

### Field of the Invention

The present invention is in the field of animal feeding and more particularly directed to producing, via gas fermentation of microorganisms, nutritional products including highprotein foods, food additives, and other products using waste gas streams from industrial processes to supply carbon and energy.

### Background of the Invention

Gas feedstocks represent a vast resource of carbon and energy that can be used to grow chemoautotrophic microorganisms for use in human and animal nutritional products. Chemoautotrophic bacteria are capable of capturing and metabolizing carbon from inorganic sources, such as CO₂, CO, CH₄, as well as, in some cases, hydrogen (H₂) or methane (CH₄) as a primary or additional source of energy. By converting inorganic carbon to organic carbon via metabolic carbon fixation, these microbes can serve as primary producers in natural environments. Many of these chemoautotrophic species can be cultured on gas feedstocks in bioreactors for commercial production of biomass, which can be processed into nutritional products such as animal feed, companion animal feed, or even food for humans, and chemicals.

Bacteria from the genera *Bacillus, Lactobacillus, Bifidobacterium* and others, which are known to be of nutritional, and/or medicinal and/or probiotic benefit are not capable of growing on gas substrates, such as CO₂, H₂, CO and CH₄. However, certain strains of chemoautotrophic microbes from the genera *Cupriavidus, Rhodobacter, Methylobacterium, Methylococcus, Rhodospirillum,* and *Rhodopseudomonas* are known to grow on gaseous substrates, such as CO₂, H₂, CO and CH₄.

Photoautotrophic microbes are bacteria, cyanobacteria, and algae that can fix carbon from CO₂ by utilizing light energy. However, certain species of photoautotrophic bacteria also exhibit chemoautotrophic metabolism, in that they are able to utilize hydrogen as an energy source to drive the fixation of CO₂ without light. Examples of these are the genera *Rhodobacter, Rhodospirillum,* and *Rhodopseudomonas.* Many other such species are known in the literature. C. *necator* has been shown to grow faster and more efficiently on CO₂/H₂ mixtures than typical acetogens that are used in some anaerobic gas fermentations.

In order to grow bacteria chemoautotrophically it is necessary to create favorable conditions in which the population will grow by employing a chemoautotrophic metabolic pathway. This includes providing a chemical substrate to supply carbon, such as CO₂, and providing a chemical substrate to supply energy such as H₂, in addition to the other requisites needed for microbial cultivation such as an aqueous environment, chemical nutrients such as, but not limited to, ammonia and phosphate and in some cases other gases such as oxygen. In some cases the carbon and energy are supplied by a single molecule such as methane. Other aspects of the chemoautotrophic environment or conditions may be common to many types of fermentation such as maintaining specific temperatures or pH, though these may be particularly tuned to best support the chemoautotrophic cultivation of different microbe strains.

Bacteria grown chemoautotrophically express different amounts (and in some cases, different types) of proteins, enzymes, transporters, fats, oils, vitamins, co-factors and other biochemicals, than are expressed by the same bacteria when grown in traditional fermentation. They may also express different levels and ratios of amino acids. Examples of this includes cytochromes, quinones (e.g., coenzyme Q), RuBisCO (Ribulose-1,5-bisphosphate carboxylase oxygenase). Because of this, bacteria grown chemoautotrophically can provide different nutritional profiles than the same bacteria grown heterotrophically. These growth differences can be advantages for providing additional vitamins, minerals, cofactors, etc. to the biomass product.

Bacteria grown chemoautrophically can also have different secretory activity, which can affect the content of the fermentation mix. Chemoautotrophic bacteria (and photoautotrophic bacteria grown chemoautotrophically) release chemicals into the growth medium, such as glycolates, polysaccharides, proteins, amino acids, fats, oils, hydrocarbons, nucleic acids, organic acids, polyhydroxyalkanoates, phasins, carotenoids, vitamins, gene transfer agents (GTA) and other biomolecules that can then be utilized by non-autotrophic bacteria and other heterotrophic microorganisms as growth substrates and growth regulators.

In industrial biotechnology, consortia are usually crafted to improve the growth of a target species within a consortium, or to remove or remediate toxic by-products produced by a target species in order to improve or aid in the growth of that particular target species. This is the case with commercial production of the target microbe, *Methylococcus capsulatus,* when grown on a substrate of methane. Because *M. capsulatus* secretes acetate during the fermentation process, and acetate inhibits growth, an additional species is added to remove the acetate, creating a consortium. However, the additional species is not a significant contributor to the final nutritional profile of the end product.

A consortium of bacteria that can increase the amounts of desirable fatty acids, vitamins, or amino acids in the final biomass product would therefore be highly desirable.

### SUMMARY

The invention is defined in claims 1, 9, 10 and 11. Particular embodiments are set out in the dependent claims

The present specification describes products, materials, intermediates, and other biological products that are produced from the waste gases of industrial processes by cultivating a microbial consortium on these gas streams. Such waste gases may include CO₂, CO, CH₄, and H₂, thereby reducing environmental pollution while at the same time saving energy and chemical feedstocks. Other trace gases which may be present in the industrial effluent, such as H₂S or SO₂, can provide additional nourishment to the autotrophic primary producer bacteria, and removal of such gases provides the added benefit of remediating these greenhouse gases from the gas stream.

In some exemplary processes the waste gas may be scrubbed of contaminants such as mercury, or other toxic compounds by passing through a bed or column of adsorbent or absorbent material or other technique for removal of which many are known and industrially practiced. In some cases the waste gas may be cooled to cause steam to condense into water so it may be removed. In some cases CO₂ in the waste gas may be concentrated by collection and release by an MEA (monoethanolamine) or another adsorbent, or by a reversable conversion to carbonate, or by carbonate in a system which comprise carbonic anhydrase enzymes, or separated via selectively permeable membranes, or fractionally distilled, or processed by any other method which would lead to a removal of other gases, an increase in CO₂, or both, prior to being fed into the bioreactor in order to increase the CO₂ concentration, reduce the concentration of non-CO₂ gases such as but not limited to nitrogen, argon, oxygen, and others.

In some exemplary processes, the waste gas, or separated components of the waste gas are introduced into a bioreactor containing a consortium of microbes that utilize the waste gas components to produce a desired product. The desired product (biomass, nutraceutical, protein, etc.) is recovered from the aqueous phase in a separate vessel or vessels, utilizing a suitable recovery process for the given product. Examples of recovery processes include extraction, distillation, and combinations thereof. The bacteria are removed from the aqueous phase and recycled to avoid toxicity and to maintain high cell concentrations, thus maximizing reaction rates. Cell separation, if desired, can be accomplished by centrifugation or membranous ultrafiltration, for example.

Examples of products that can be produced by the methods disclosed herein include biomass, feed ingredients, proteins, vitamins, probiotics, natural antibiotics, and organic acids. These can be produced from the waste gas streams of industrial processes such as brewing, bioethanol production, cement manufacturing, oil refining, and similar processes that generate waste CO₂ and/or H₂. Preferably these products are produced from waste gas streams by continuous gaseous substrate fermentation, under aerobic conditions.

In various embodiments, chemoautotrophic microbes are employed and the primary sources of both carbon and energy to the microbes are both supplied as gases, such that the primary producers support a defined consortium of bacterial species via mutualistic interactions. A consortium of defined species in the present invention, after a period of cultivation on suitable gases, can be used as a nutritional product. To make improved nutritional products, multiple different species are cultivated together, such that each provides different nutritional properties. The use of multiple microbes allows for controlled modification and customization of the nutritional composition of the final product. It is also possible to include one or more genetically modified strains which produce a key component, such as astaxanthin, a carotenoid which is a key ingredient for aquaculture feed products.

An advantage of the present invention is that the dried biomass can be blended into an aquafeed or other nutritional product to replace the fishmeal that is normally harvested and used for feeding farmed fish and seafood, such as salmon, trout, tilapia, and shrimp. Replacing fishmeal in commercial aquaculture would reducing the stress on the world's fisheries (Pitcher & Cheung, 2013), as the demand for fishmeal is already projected to outstrip supply. Products of the present invention can also be used as a high protein food, feed or ingredient for many types of animals including pets and humans.

Another advantage of the present invention is that the composition of a consortium can be adjusted to fine-tune the nutritional composition of the biomass product. This ability is advantageous because, for example, recent studies have shown that adapting the amino acid composition of feed given to laboratory mice based on the animal's own overall amino acid composition not only reduces the amount of feed needed, but also improves the health of the mice. Also, aquaculture and other animal feeds and feed additives are altered by selecting the amounts of various ingredients added to achieve desired amino acid profiles. By adjusting the consortium, a number of applicationspecific nutritional products can be derived including for use by humans. Special consortia can be created to create products for aid in treating medical conditions, dietary deficiencies, or to be optimized for feeding a particular growth stage of a given organism.

In an alternative embodiment, one or more of the strains in the consortium may naturally, or be genetically modified to, produce a valuable small molecule (e.g., a specific fatty acid) or a protein product (e.g., an enzyme, therapeutic protein, antibiotic, hormone, vitamin, precursor, antibody, or vaccine). The present invention provides methods to produce such molecules using inexpensive gas feedstocks even if the host organism(s) cannot grow solely on gas.

In various embodiments a food or feed product is produced by cultivating microbes on gaseous substrates, the food or feed product having characteristics that provide nutritional, medical, and/or dietary benefits, where the microbes comprise a consortium of chemoautotrophic microbes, photoautotrophic and non-chemoautotrophic microbes. The consortium creates an ecosystem in which the chemoautotrophic microbes form the base of a food chain on which the non-autotrophic microbes are capable of growing

(Braakman et al, 2017). One goal of the gas-based fermentation process described herein is to generate a biomass product that has enhanced nutritional value compared to the biomass that the same chemoautotrophic microbes would produce if grown alone. A further economic benefit of this invention is to facilitate the growth of desired or beneficial microbes that otherwise could not utilize the inexpensive CO₂ as a primary carbon source, and hydrogen for energy.

A number of species of microbe are known to be nutritionally beneficial, or to produce beneficial substances, or have probiotic properties which can be significant components of a product which can be produced by this method. Examples of these are: *Aspergillus niger, Aspergillus oryzae, Bacillus coagulans, Bacillus lentus, Bacillus licheniformis, Bacillus megaterium, Bacillus pumilus, Bacillus subtilis, Bacteroides amylophilus, Bacteroides capillosus, Bacteroides ruminocola, Bacteroides suis, Bifidobacterium adolescentis, Bifidobacterium animalis, Bifidobacterium bifidum, Bifidobacterium infantis, Bifidobacterium lactis, Bifidobacterium longum, Bifidobacterium thermophilum, Bifidobacterum breve, Lactobacillus acidophilus, Lactobacillus brevis, Lactobacillus bulgaricus, Lactobacillus casei, Lactobacillus cellobiosus, Lactobacillus curvatus, Lactobacillus delbruekii, Lactobacillus fermentum, Lactobacillus helveticus, Lactobacillus johnsonii, Lactobacillus lactis, Lactobacillus paracasei, Lactobacillus parafarraginis, Lactobacillus plantarum, Lactobacillus reuterii, Lactobacillus rhamnosus, Lactobacillus salivarius, Lactobacillus sporogenes, Lactococcus lactis, Leuconostoc mesenteroides, Pediococcus acidilactici, Pediococcus cerevisiae, Pediococcus pentosaceus, Propionibacterium shermanii, Propionibacterium freudenreichii, Saccharomyces boulardii, Saccharomyces cerevisiae, Streptococcus cremoris, Streptococcus diacetylactis, Streptococcus faecium, Streptococcus intermedius, Streptococcus lactis, Streptococcus thermophiles.*

According to a first aspect of the invention a method is provided for producing a biomass, the method comprising: providing a gas mixture to a consortium of bacteria and microbes in a bioreactor, the consortium including at least three of Cupriavidus necator, Rhodobacter sphaeroides, Rhodopsuedamonas palustris, Rhodobacter capsulatus, Bacillus subtilis, and Bacillis magaterium, the gas mixture including each of hydrogen gas, carbon dioxide gas, and oxygen gas, whereby hydrogen is metabolized by at least some of the bacteria of the consortium and growth of the consortium occurs solely by the fixation of carbon dioxide; harvesting cells of the bacteria and microbes from the bioreactor; and drying the solids to yield the biomass.

In an embodiment of the first aspect the method further comprises separately receiving hydrogen gas and flue gas, the flue gas including both the carbon dioxide gas and the oxygen gas, and mixing the hydrogen gas and flue gas to produce the gas mixture. In particular the mixing the hydrogen gas and flue gas comprises diluting the flue gas approximately 5-fold with the hydrogen gas.

In an embodiment of the first aspect one of the hydrogen gas, carbon dioxide gas, and oxygen gas is introduced separately into the bioreactor from the other two gases.

In an embodiment of the first aspect the consortium of bacteria consists solely of Cupriavidus necator, Rhodobacter sphaeroides, Rhodopsuedamonas palustris, Rhodobacter capsulatus, and Bacillus subtilis.

In an embodiment of the first aspect at least one of the bacterium of the consortium is of a strain that has been adapted over multiple generations to be tolerant of a flue gas, and wherein the gas mixture includes the flue gas.

In an embodiment of the first aspect the method further comprises, before providing the gas mixture to the consortium, adapting a bacterium of the consortium to a flue gas by growing multiple successive generations of the bacterium in the presence of the flue gas to produce an adapted bacterium, wherein the gas mixture includes the flue gas.

In an embodiment of the first aspect one of the bacterium of the consortium expresses a carotenoid.

According to an embodiment a product is made by the method according to the first aspect and its embodiments.

According to a second aspect of the invention a method is provided for producing a feed product comprising: producing a biomass by providing a gas mixture to a consortium of bacteria and microbes in a bioreactor, the consortium including at least three of Cupriavidus necator, Rhodobacter sphaeroides, Rhodopsuedamonas palustris, Rhodobacter capsulatus, Bacillus subtilis, and Bacillis magaterium, the gas mixture including each of hydrogen gas, carbon dioxide gas, and oxygen gas, whereby hydrogen is metabolized by at least some of the bacteria of the consortium and growth of the consortium occurs solely by the fixation of carbon dioxide; harvesting cells of the bacteria and microbes from the bioreactor; and dewatering, concentrating or drying the solids to yield the biomass; and blending the biomass with an additive.

According to a third embodiment a food or feed additive is provided, comprising: a consortium including Cupriavidus necator, a species from the genus Rhodobacter, a species from the genus Rhodopseudomonas, and a species from the genus Bacillus.

In an embodiment of the third aspect the species from the genus Rhodobacter comprises Rhodobacter sphearoidies.

In an embodiment of the third aspect the species from the genus Rhodopseudomonas comprises Rhodopseudomonas palustris. In particular the species from the genus Rhodobacter comprises Rhodobacter sphearoidies. More specifically the genus Bacillus comprises bacillus subtilis.

In an embodiment of the third aspect the food or feed additive comprises Rhodobacter capsulatus.

In an embodiment of the third aspect the food or feed additive further comprises Rhodobacter capsulatus.

In a specific embodiment of the third aspect the food or feed additive the species from the genus Rhodopseudomonas comprises Rhodopseudomonas palustris, in particular the species from the genus Rhodobacter comprises Rhodobacter sphearoidies, further in particular the species from the genus Bacillus comprises bacillus subtilis, further in particular the food or feed additive comprises Rhodobacter capsulatus.

In a specific embodiment of the third aspect the food or feed additive the species from the genus Rhodopseudomonas comprises Rhodopseudomonas palustris, in particular the species from the genus Rhodobacter comprises Rhodobacter sphearoidies, further in particular the food or feed additive comprises Rhodobacter capsulatus.

In an embodiment of the third aspect the consortium is characterized by an enhanced attractiveness to fish.

In an embodiment of the third aspect the consortium is characterized by an enhanced palatability to fish.

In an embodiment of the third aspect the C. necator comprises over 85% of a dry weight of the consortium of the food or feed additive.

### BRIEF DESCRIPTIONS OF THE DRAWINGS

- FIG. 1: is a schematic representation of an exemplary bioreactor.
- FIG. 2: is a bright field micrograph of a consortium grown on CO₂, H₂, and O₂ showing multiple species of microbes.
- FIG. 3: is a tabular comparison of amino acid profiles between an exemplary final product according to an embodiment and three products of the prior art.
- FIG. 4: is a tabular comparison of amino acid profiles between the exemplary final product of FIG. 3 and data *Bacillus subtilis* grown heterotrophically on sugars.
- FIG. 5: is a graphical representation of weight gain per period for fish fed either an exemplary final product or a fishmeal control diet.
- FIG. 6: is a graphical representation of total weight gain for fish fed either the exemplary final product or the fishmeal control diet of FIG. 5.

### DETAILED DESCRIPTION

### Microbial strains.

*C. necator* has the advantage that it can grow very rapidly and to high density on a mixture of H₂, CO₂, and O₂, and it can be continuously cultured for long periods of time without contamination. Bacterial strains for the consortium are selected for ability to grow on H₂ and CO₂, being naturally-occurring (i.e., non-GMO), generally recognized as safe ('GRAS') organisms, or because of their apparent beneficial qualities and apparent lack of negative characteristics, so that they will be broadly suitable for feed and food processing, although GMO organisms designed for a specific purpose (e.g., metabolite production via an engineered pathway) can also be included, if desired.

Experimental results for exemplary products are discussed herein. Strains used for such experimental results were obtained as pure, axenic type-cultures from culture collections. Further selection was made based on a series of experiments where different bacteria of interest were added to a consortium which was then grown on gas, and after a period of time, subjected to metagenomic analysis via the 16s technique to identify species presence and amount in the final product. These final products we subjected to further analyses including feeding trials with live animals to determine digestibility, palatability, and whether anti-nutritional properties were present. During this work the final products were found to be nutritionally adequate or superior to existing high protein feed and food additives. Surprisingly, products produced through the cultivation of the disclosed consortia also exhibited much higher palatability in aquaculture diets than the industry standard high protein ingredient, fishmeal. The products were also found to be more attractive to fish, and fish ate it at an increased feed consumption rate.

### Gas supply.

The CO₂, H₂ and O₂ can be supplied from either flue gas collected from an industrial emitter (designated as 'flue gas') or from pure stocks of compressed gas obtained from a gas supplier (designated as 'lab gas'). These gases can also be derived from process gas produced by an industrial process, from gasifier or pyrolysis output gas, from syngas, from electrolysis of water, from a steam methane reformer, via separation from air, from the manufacture of cement or lime such as quicklime, from a combustion process, or from any industrial, natural, or other process which produces one or more of the desired gases.

An example of CO₂ produced by an industrial process is the production of CO₂ during the manufacture of cement or quicklime where limestone (CaCO₃) is heated to form CaO and CO₂, in a process sometimes called calcination. For production of feed, food, nutraceuticals, biologicals, and the like, an industrial source of waste CO₂ that is free of toxic elemental contaminants (e.g., mercury) is preferred. Examples of such sources include CO₂ from breweries and bioethanol plants. Hydrogen can be supplied as part of the gas composition of pyrolysis gas, syngas, as an industrial side product from activities such as propylene manufacture, as a component of a mixed gas stream from an oil refinery, or in gas created by steam methane reformation (SMR) process, from compressed gas, or from electrolysis of water. Oxygen can be obtained from atmospheric gas, as a product of electrolysis, as a by-product of ammonia manufacture, or as a component of industrial by-product gas such as cement flue-gas.

In some embodiments, where the CO₂ and O₂ are derived from either lab gas or flue gas, these gases are further diluted approximately 5-fold with H₂ to supply bacteria with a feedstock mixture that is optimized for growth. For a commercial-scale operation, the fermentation plant can be located near the gas production site, or the gas can be transported by vehicle or pipeline to the biomass production site.

For injection into the fermenter, in the experimental processes, the gas supply was filtered through 0.2 um filters to remove particles and microorganisms. For these experiments, compressed H₂, CO₂ and O₂ were each regulated to 20 psi. The gases were delivered to a flow proportioner, which set the relative fraction of the gases, and to a variable area flow meter to control the mixture and flow rate into the fermenter.

### Nutrient monitoring.

The compositions of the input and output gases can be measured and monitored to determine the gas uptake rates, the mass balances, and the mass transfer efficiency for dissolution of the gas into the solution and the biomass. Key nutrients (such as NH₄, PO₄ and SO₄), can also be monitored and replenished to prevent nutrient limitations that might restrict bacterial growth.

### Microbial inocula.

The inocula for fermenter runs can be prepared in many ways; each microbial strain may be grown separately, or two or more may be grouped together in a single fermentation. Heterotrophic species are always grown up from pure cultures on an heterotrophic medium that is suitable for propagating the particular species (or group of species) being grown. Chemoautotrophic species can be grown on gases, or, in some cases, on heterotrophic media. Photoautotrophic species may be grown using light or heterotrophic media. Some photoautotrophic species are also chemoautotrophic, and thus may be grown on gaseous substrates. In various embodiments inocula comprise axenic strains of each microbe, that is, they are cultivated in a closed bioreactor where other microbes are kept out such that the final product contains just these bacteria. Inoculating the bioreactor can further involve the sterile addition of a culture containing one or more inocula into the bioreactor.

In some embodiments, all of the cultures for the consortium are added to the bioreactor, in a short period of time, at the beginning of the fermentation procedure or run. In other embodiments, chemoautotrophic microbes are added to the bioreactor at the beginning of the fermentation, and the inoculum cultures containing other species are added at later points. In further embodiments, the timing of the addition, amount and density of culture additions, and method of preparing inocula can be altered to affect the qualities, composition, and/or value of the final product. In some embodiments, additional inoculations of one or more strains used in the consortia can be added at later times.

In the experimental runs, cultures were prepared by growing *C*. *necator* and the other chemoautotrophic species on H₂/CO₂/O₂ to an OD620 ~1 in small bottles of media equipped with gas fittings, or on heterotrophic media such as YT, LB, or on minimal salts media supplemented with an organic carbon source such as sugars, gluconate, glycerol or organic acids. Non-chemoautotrophic species were grown in liquid yeasttryptone medium (YT medium), a well-known and commercially available medium. The bioreactor was inoculated to OD -0.1. A ca. 5% inoculum is ideal. The pH was controlled with 2N NH₄OH, or with NH₄OH when ammonia is also desired in order to supply nitrogen. Fermentation runs were carried out over a period of days, resulting in OD620 of 1-100 or greater.

The recovered biomass was analyzed for protein and lipid content and the composition of each product. Proprietary strains of *C*. *necator* and/or *R. capsulatus,* or other microbes were sometimes used in addition to type strains. Several of these proprietary strains of chemoautotrophic species are adapted to flue gas and therefore tolerant to various toxic gas components, which can be included in some complex industrial flue gases. In some cases, additional inoculations with one or more of the consortium strains were carried out at later times.

Table 1 provides a consortium disclosed in U.S. Patent Application No. 15/641,114. Cultivation of this consortium generates biomass which yielded favorable nutritional analysis and performed well in field studies with rainbow trout that were conducted by the US Fish and Wildlife Service, Bozeman Montana Fish Technology Center.

**Table 1**

| Strain | Species | Source |
|---|---|---|
| B-3226 *Rhodospirillum rubrum,* (ARS NRRL Type Strain) | | |
| B-1727 *Rhodobacter sphaeroides,* (ARS NRRL Type Strain) | | |
| B-4276 *Rhodopseudomonas palustris,* (ARS NRRL Type Strain) | | |
| B-14308 *Bacillus megaterium,* (ARS NRRL Type Strain) | | |
| B-356 *Bacillus subtilis,* (ARS NRRL Type Strain) | | |
| B-354 *Bacillus subtilis,* (ARS NRRL Type Strain) | | |
| B-14200 *Bacillus subtilis* subspecies *subtilis,* (ARS NRRL Type Strain) | | |
| B-41406 *Bifidobacterium animalis* subspecies *animalis,(ARS* NRRL Type Strain) | | |
| B-4495 *Lactobacillus acidophilus,* (ARS NRRL Type Strain) | | |
| B-1922 *Lactobacillus casei* subspecies *casei,* (ARS NRRL Type Strain) | | |
| B-4383 *Cupriavidus necator,* (ARS NRRL Type Strain) | | |
| B-14690 *Cupriavidus necator,* (ARS NRRL Type Strain) | | |
| *Cupriavidus necator* strain H16 (ATCC Type Strain) | | |
| *Rhodobacter capsulatus* strain SB-1003 (ATCC Type Strain) | | |
| OB213 *Rhodobacter capsulatus,* Oakbio Proprietary Strain | | |
| OB311 *Cupriavidus necator,* Oakbio Proprietary Strain | | |

Surprisingly, a metagenomic analysis (conducted by Zymo Research, Irvina, CA) showed that the majority of the biomass product was composed of a subset of the original species of the consortium. Subsequent experiments confirmed this.

Table 2 provides exemplary microbes for use in combinations according to various consortia embodiments.

**Table 2**

| Strain | Species | Source |
|---|---|---|
| B-1727 *Rhodobacter sphaeroides,* (ARS NRRL Type Strain) | | |
| B-4276 *Rhodopseudomonas palustris,* (ARS NRRL Type Strain) | | |
| B-14308 *Bacillus megaterium,* (ARS NRRL Type Strain) | | |
| B-356 *Bacillus subtilis,* (ARS NRRL Type Strain) | | |
| B-354 *Bacillus subtilis,* (ARS NRRL Type Strain) | | |
| B-14200 *Bacillus subtilis* subspecies *subtilis,* (ARS NRRL Type Strain) | | |
| B-4383 *Cupriavidus necator,* (ARS NRRL Type Strain) | | |
| B-14690 *Cupriavidus necator,* (ARS NRRL Type Strain) | | |
| H16 *Cupriavidus necator* strain H16 (ATCC Type Strain) | | |
| SB-1003 *Rhodobacter capsulatus* strain (ATCC Type Strain) | | |
| OB213 *Rhodobacter capsulatus,* Oakbio Proprietary Strain | | |
| OB311 *Cupriavidus necator,* Oakbio Proprietary Strain | | |

Consortia are not required to include all of these strains, and additional microbes not listed can be employed as well. A typical fermentation can thus comprise, for example, microbes from each of:
*Cupriavidus necator*
*Rhodobacter sphaeroides*
*Rhodopseudomonas palustris*
*Rhodobacter capsulatus*
*Bacillus subtilis.*

### Bioreactor Fermentation.

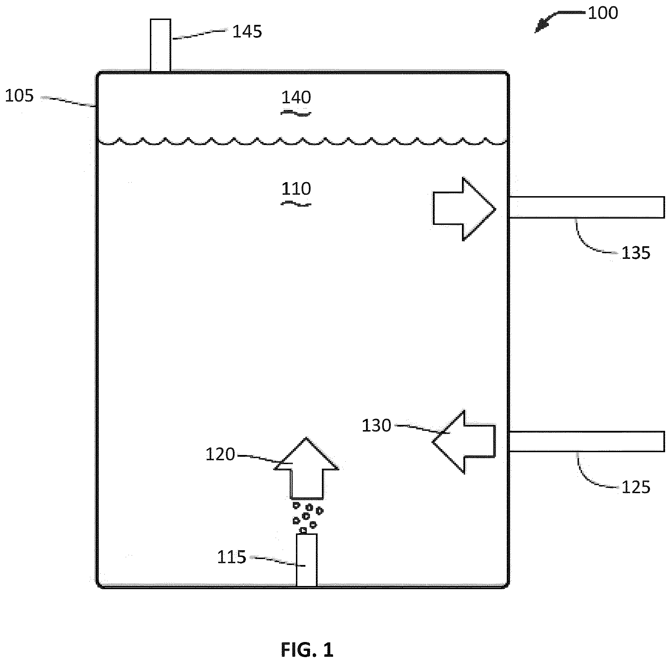

In various embodiments a bioreactor for chemoautotrophic synthesis can be used, though many types and designs of bioreactor are suitable. For the cultivation of the product discussed herein a suitable bioreactor includes a vessel at least partially filled with a liquid medium in which the microbes are dispersed. The liquid medium comprises chemicals required for growth of the microbes, examples of which are described below. At least one port exists for introducing the gaseous substrates into the liquid in the bioreactor. The vessel may have a headspace into which gases collect after traversing the fluid in the vessel. An exhaust port allows gases to exit the vessel. Additional ports are present as needed for sensors, addition of liquids or chemicals and removal of product, liquids, or samples for testing, as would be expected to be found on common bioreactors, which are well known in the field of fermentation, cell culture and microbe cultivation. A minimal design bioreactor is shown in FIG. 1.

In FIG. 1, bioreactor 100 includes a vessel 105 that in operation holds a quantity of a liquid medium 110 containing the chemoautotrophic micro-organisms and other microbes in culture. The bioreactor 100 also includes a substrate port 115 through which a gaseous substrate 120 can be introduced into the vessel 105 for introduction into the liquid medium 110, a media inlet port 125 through which fresh media 130 can be introduced into the vessel 105 for introduction into the liquid medium 110, and a media outlet port 135 through which the medium 110 can be removed, for example, to harvest biomass and/or chemical products. The bioreactor 100 can also comprise a headspace 140 and a gas release valve 145 to vent gases from the headspace 140. In some embodiments, the media outlet port 135 and the media inlet port 125 are connected via a system which harvests biomass and reconditions the medium 110 for recirculation. In some embodiments, the gas release valve 145 is attached to a system which re-circulates the gaseous substrate back to the substrate port 115, and may make additions or subtractions to optimize the gas composition.

For the purposes of the experimental results described herein, bioreactors comprised custom-built 250ml, 1L, and 4L, glass flask-based bioreactors or a commercially manufactured New Brunswick Scientific Bio Flo 4500 with the 4-gas handling option. Some experiments were conducted in 5L and 55L looping gas bioreactors. Fermentations were run at a constant or varied temperatures between 15 and 70°C, for example at 30°C. Additional bioreactor designs that can be used in conjunction with the present invention can be found in U.S. Patent Application Serial No. 13/204,649 filed on August 6, 2011 and entitled "Chemoautotrophic Bioreactor Systems and Methods of Use" which is incorporated herein by reference. Bioreactors for various embodiments can comprise one or more vessels and/or towers or piping arrangements, and can comprise, for example, a Continuous Stirred Tank Reactor (CSTR), an Immobilized Cell Reactor (ICR), a Trickle Bed Reactor (TBR), a Bubble Column, a Gas Lift Fermenter, a Static Mixer, a Fluidized Bed, an Up-flow or Down-flow, a continuous, batch or loop reactor, or any other vessel or device suitable for maintaining suitable gasliquid contact. In some embodiments, the bioreactor may comprise a first growth vessel and a second chemoautotrophic synthesis vessel, while in other embodiments a single vessel is used throughout both of the growth and synthesis stages.

In some embodiments, a gas recirculation system can be used to improve the conversion efficiency, particularly during a continuous process, in order to reduce the total gas requirement. Continuous harvesting of the cell mass can be advantageous for commercial production processes, and can be implemented through the continuous removal of cell broth and the continuous replenishing of medium, in order to maintain the culture volume and cell density.

### Monitoring cell growth and species diversity.

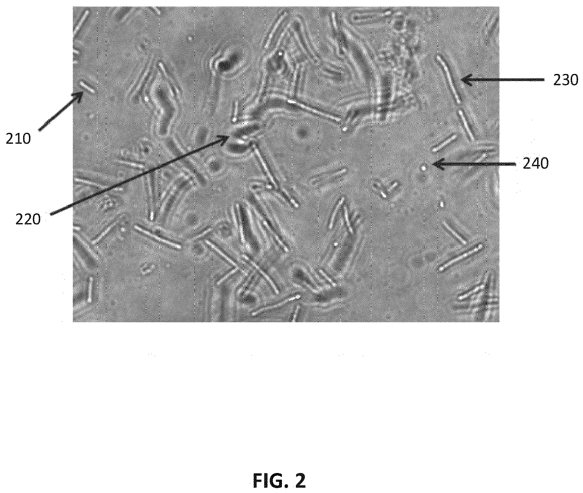

To monitor the progress of cell growth and verify the species diversity of a culture, samples can be periodically removed for analysis, or the bioreactor system can comprise analytic equipment. Removal allows for characterization techniques such as microscopy to determine cell morphology, an example of which is shown in FIG. 2 (from the completion of a run), a brightfield micrograph showing that the species diversity was maintained. Four different species are numbered 210, 220, 230, and 240 in the micrograph. Species diversity can also be monitored and quantified using methods well known in the art, such as analysis of 16S rRNA genes, 23S rRNA genes, or other genetic markers and phenotypic indicators (Jovel et al., 2016). Metagenomic analysis of exemplary fermentation products was carried out by Zymo Research, Irvine, CA. Growth behavior was characterized by optical density (OD) measurements at 620 nm using an ICN TiterTek 96-well plate reader. Aliquots of each fermenter sample (200 ul) were measured in duplicate to plot the growth curves.

### Carbon capture.

Carbon capture from a new source of flue gas can be verified by performing headspace gas analysis, as well as growth experiments that use the flue gas as the sole carbon source for bacterial biomass production. The dry weight of each culture can also be determined by centrifuging the culture, washing the pellet, drying the cells in a lyophilizer, and weighing the lyophilized cells.

### Gas mixing.

For hydrogen fermentations typically, the CO₂ feedstock or raw flue gas can be diluted with hydrogen gas to achieve ratios of about 8:1 to 1:1 (H₂:CO₂, v/v), resulting in a final CO₂ concentration of about 50% - 1% or less. The O₂ concentration is ideally 3-15%, or 3-12%, or 8-15%, or 8-12%. For methane fermentations, typically the methane concentration is between 80% to 5%, CO₂ is between 40% - 1%, and the O₂ concentration is 50% to 5%. In either system, CO can be up to 10%, and a variety of other gases may be present, including sulfur oxides, nitrogen oxides, hydrogen sulfide, molecular nitrogen or other gases found in the gas source.

### Culture medium.

Many different mineral media recipes can be used, and varying the media is one of the ways the characteristics of the final product can be influenced. An exemplary mineral salts medium (modified from Repaske & Mayer, 1976) containing no organic carbon or complex nutrients comprises Na₂HPO₄·2H₂O 4.5 g/L, KH₂PO₄ 1.5 g/L, NH₄Cl 1.8 g/L, MgSO₄·7H₂O 0.11 g/L, NaHCO₃ 0.2g/L, FeSO₄·7H₂O 12 mg/L, CaCl₂·2H₂O 10 mg/L, ZnSO₄·7H₂O 100 µg/L, MnCl₂·4H₂O 30 µg/L, H₃BO₃ 300 µg/L, CoCl₂·6H₂O 200 µg/L, CuCl₂·2H₂O 10 µg/L, NiCl₂·6H₂O 20 µg/L, Na₂MoO₄·2H₂O 30 µg/L. Systems operating at larger volumes for commercial production can employ different compositions, for example, less phosphate may be required as an active Ph control, limiting the need for a strong phosphate buffer system.

### Concentration and harvesting.

Biomass products can be harvested through many methods, such as filtration, gravity separation, or other methods, of which many are industrially practiced. Drying can be achieved by spray drying, freeze drying, thermal drying, desiccation, or many other methods, many of which are currently practiced industrially.

Heat treatment, radiation exposure, or chemical treatment can be useful if the cells must be made non-viable prior to further processing. The dried material can be easily blended with other ingredients to form a nutritious fish feed that can replace aquafeed products that typically rely on fishmeal for protein, fatty acids, and other nutrients. Exemplary additives that can be blended with the dry material include oils, plant-based ingredients, animal-based ingredients such as fishmeal, feather meal, blood meal, minerals, vitamins, amino acid supplements, attractants, colorants, medicines, flow additives, and preservatives. The amino acid composition of the dried material from a 30L batch of the cultivated consortium shown in Table 2 compares favorably to that of fishmeal (IAFMM Report, 1970), in that it has similar amino acid distribution.

Hot water, enzymatic, protein isolation or other treatments may be used to reduce the amount of nucleic acids in the material. Recovered cellular biomass can be subjected to lysis by heat, chemical, enzymatic or mechanical disruption such as freeze thaw, pressure cell, grinding, or shear, to obtain a protein isolate.

In the below referenced example, cell suspensions were removed from the fermenter via a sterile exit port. The supernatant was then removed by centrifugation to form a cell pellet. The cells were then washed in a low-salt buffer solution, and then re-pelleted. The final cell paste was then freeze dried to a powder using a commercial Labconco lyophilizer.

A cultivation of the consortium shown in Table 2 was performed at the 55L scale in a custom-built looping gas bioreactor. The cultivated product consisted of chemoautotrophs, photoautotrophs, and probiotic heterotrophs, Amino acid composition analysis was conducted by NP Analytical Laboratories (St. Louis, MO) and shown below in Table 3:

**Table 3**

| Amino Acid | g/100 g Dried Biomass |
|---|---|
| Aspartic Acid | 4.49 |
| Threonine | 2.40 |
| Serine | 1.79 |
| Glutamic Acid | 7.31 |
| Proline | 1.67 |
| Glycine | 2.15 |
| Alanine | 3.24 |
| Valine | 3.34 |
| Methionine | 1.16 |
| Isoleucine | 2.60 |
| Leucine | 3.24 |
| Tyrosine | 1.71 |
| Phenylalanine | 1.99 |
| Histidine | 0.930 |
| Lysine | 3.48 |
| Arginine | 2.45 |
| Cysteine | 0.312 |
| Tryptophan | 0.519 |

At the conclusion of fermentations with the consortia mentioned in Table 2, beginning with all 12 species of bacteria, a metagenomic analysis carried out by Zymo Research, Irvine, CA on the final products. Surprisingly, it was found that the bacterial population in the final product represented a subset of the initial species provided. An exemplary final species composition comprised 92.10% *C. necator,* 4.30% *Rhodobacter,* 0.20% *Bacillus,* and a trace amount of *Phodopseudamonas.* Another exemplary final composition, starting from the same consortium but using a larger gas-fed bioreactor, had a final species composition comprising 93.70% *C. necator,* 0.30% *Rhodobacter,* 0.20% *Bacillus,* and 5.80% others.

Accordingly, various embodiments of consortia consist of, or consist essentially of, at most six species of bacteria and microbes and in some cases only 3. These six species consist of *Cupriavidus necator, Rhodobacter sphaeroides, Rhodopsuedamonas palustris, Rhodobacter capsulatus, Bacillus subtilis,* and *Bacillis magaterium.*

In the various experimental final products *C*. *necator* comprised in excess of 85% of the total microbial population, independent of which strain of *C*. *necator* was in the initial consortium. Likewise, the use of different *B. subtilis* strains did not have a significant impact on the nutritional composition of the final product or the overall representation of *Bacillus* in the final product which was always below 5%.

In further experiments, when consortia were grown on gas, as described above, from starting consortia comprising *C*. *necator, R. sphaeroides, R. palustris,* and *B. subtilis,* each of these microbes were represented in the final product again with *C*. *necator* comprising over 85% of the final product and *B. subtilis* comprising less than 5%. One exemplary final product consisted of 86.10% *C. necator,* 2.40% *Rhodobacter,* 0.30% *Bacillus,* and trace *Rhodopseudomonas.*

An exemplary food or feed additive according to some embodiments, comprises, consists of, or consists essentially of a biomass including *Cupriavidus necator,* a species from the genus *Rhodobacter,* a species from the genus *Rhodopseudomonas,* and a species from the genus *Bacillus.* In various embodiments the species from the genus *Rhodobacter* comprises *Rhodobacter sphearoidies,* and/or the species from the genus *Rhodopseudomonas* comprises *Rhodopseudomonas palustris,* and/or the species from the genus *Bacillus* comprises *bacillus subtilis.* Any of the exemplary foods or feed additives noted above can additionally comprise, consist of, or consist essentially of that biomass plus *Rhodobacter capsulatus.* These compositions are characterized by an enhanced attractiveness to fish and/or characterized by an enhanced palatability to fish.

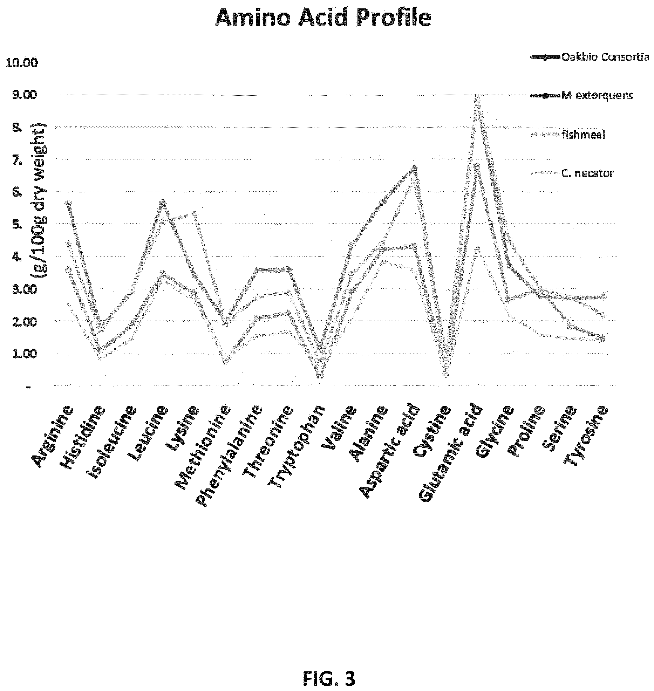

Table 4, plotted in FIG. 3, provides a comparison of amino acid profiles between an exemplary final product from the chemoautotrophic cultivation of a consortium comprised of four species of bacteria, *C*. *necator, R. palustris, R. sphearoidies* and *B. subtilis* and three other products of the prior art, *M. extorquens,* fishmeal, and *C*. *necator.* The exemplary final product included 86% total protein, *M. extorquens* comprised 53% total protein, the fishmeal comprised 70% total protein, and *C*. *necator* comprised 41% total protein. The *C*. *necator* was a monoculture also grown chemoautotrophically by the applicant. The data for *M. extorquens* is drawn from the GRAS notice AGRN 33 filed on August 16, 2019 with the USDA. Amino acid profiles for the exemplary and *C*. *necator* products were performed by Nestle Purina Analytical labs, St. Louis, MO. the Fishmeal and *M. extorquens* are currently approved for use in aquaculture diets.

**Table 4**

| AA | Amino Acid | Experimental product | *M extorquens* | fishmeal | *C*. *necator* |
|---|---|---|---|---|---|
| Arg | Arginine | 5.64 | 3.59 | 4.38 | 2.546 |
| His | Histidine | 1.79 | 1.08 | 1.69 | 0.822 |
| Ile | Isoleucine | 2.91 | 1.87 | 2.97 | 1.45 |
| Leu | Leucine | 5.66 | 3.46 | 5.08 | 3.29 |
| Lys | Lysine | 3.42 | 2.87 | 5.30 | 2.635 |
| Met | Methionine | 2.00 | 0.76 | 1.91 | 0.89 |
| Phe | Phenylalanine | 3.56 | 2.10 | 2.75 | 1.543 |
| Thr | Threonine | 3.60 | 2.25 | 2.89 | 1.682 |
| Trp | Tryptophan | 1.15 | 0.29 | 0.71 | 0.614 |
| Val | Valine | 4.36 | 2.90 | 3.46 | 2.1 |
| Ala | Alanine | 5.69 | 4.21 | 4.45 | 3.85 |
| Asp | Aspartic Acid | 6.75 | 4.32 | 6.42 | 3.581 |
| Cys | Cysteine | 0.77 | 0.36 | 0.56 | 0.265 |
| Glu | Glutamic acid | 8.82 | 6.77 | 8.90 | 4.32 |
| Gly | Glycine | 3.72 | 2.66 | 4.52 | 2.19 |
| Pro | Proline | 2.78 | 2.97 | 2.97 | 1.58 |
| Ser | Serine | 2.73 | 1.83 | 2.75 | 1.476 |
| Tyr | Tyrosine | 2.75 | 1.47 | 2.19 | 1.392 |

It can be seen that the experimental final product had the highest protein content, and generally higher levels of the essential amino acids that the three other products. Where the experimental final product did not exceed the others, it was at least similar, for example, cystine, proline, and lysine. Particularly, methionine is at a level of 2% of cell dry weight in the experimental final product which exceeds the level in fishmeal by a small amount but exceeds the level found in *M. extoquens* and monocultured *C*. *necator* by more than double. Methionine is an essential amino acid which is normally only found in low concentrations in plant proteins, so methionine is often added as a separate amino acid ingredient in formulated diets to compensate for its natural low level.

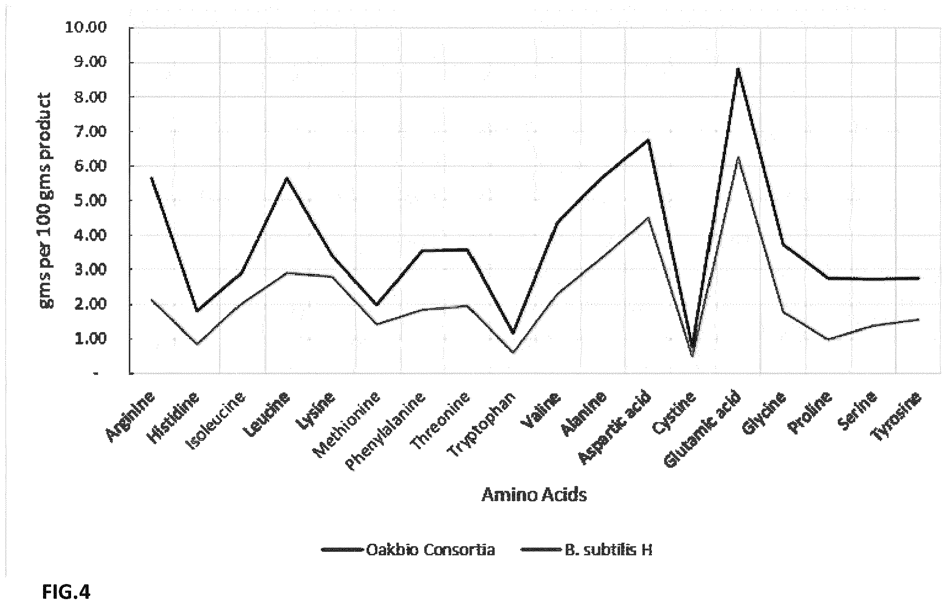

FIG. 4 shows a further comparison between the same data as above for the chemoautotrophically grown experimental final product and data for *Bacillus subtilis* grown heterotrophically on sugars. The *B. subtilis* yielded only 43% total protein, and also produced a large amount of bad smelling polyamine compounds which would make it unsuitable as a feed additive, and also unpleasant to handle.

In a test conducted by the U.S. Fish and Wildlife Service Fish Technology Center, Bozeman Montana, juvenile Rainbow Trout, (*Oncorhynchus mykiss*)*,* a commercially relevant salmonid species, were fed diets which compared the experimental final product to a high-quality commercial fishmeal. In this study a very high level of the experimental final product, 53% by weight of the formulated feed, was fed to groups of 100 juvenile salmon and their growth compared to similar groups fed a diet comprising a protein content match of high-quality fishmeal. The very high inclusion rate of the experimental final product was more than twice as high as a normal inclusion rate of fishmeal or protein, was meant to test for antinutritional properties by observing mortality during the study as well as growth.

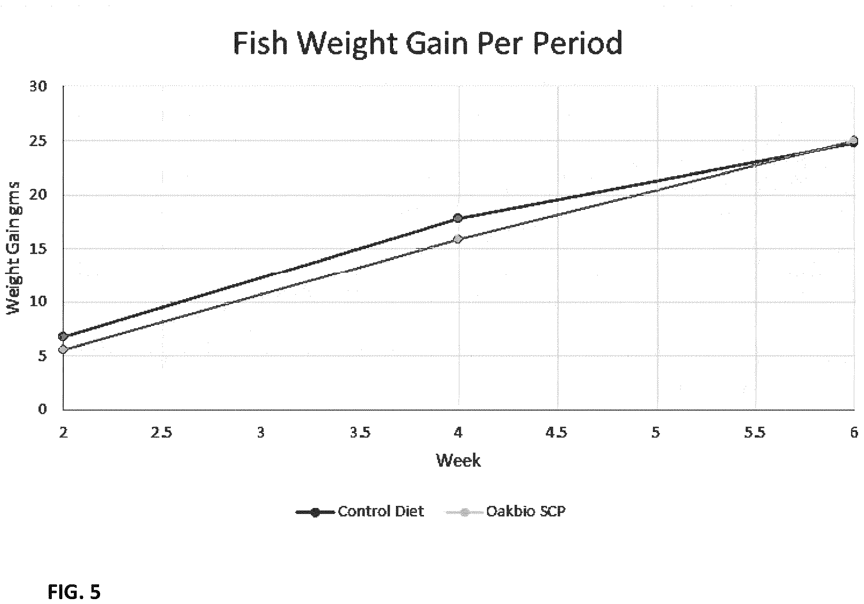

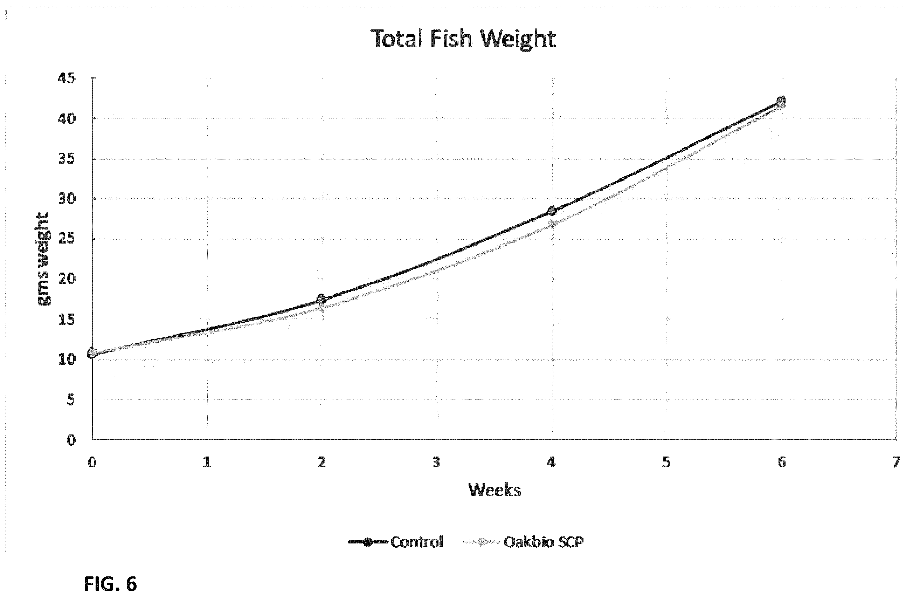

The results are illustrated, in part, by FIGs. 5 and 6. As shown in these graphs, the testing found that the fish fed the experimental final product and the fish fed the fishmeal diet were the same weight at the end of the study. The fish fed the experimental final product grew more slowly on average initially, but ultimately exceeded the growth rate of the fishmeal fed fish between weeks 4 and 6. Mortality over a 6-week trial showed statistically identical results with 99 of the 100 fish tested with the experimental final product survived to the end of the trial.

A subsequent test of digestibility and palatability was carried out by Japan Scientific Feeds Association, JFSA Report No. 2020486, March 11, 2021. For digestibility, 135 rainbow trout with an average body weight of 106.7 g were divided into 9 experimental and control groups of 15 fish each. Three control groups were fed formulated diets comprising fishmeal. The experimental diets consisted of 3 groups which were fed a diet comprising 20% of the experimental final product in place of the fishmeal, and another 3 groups which were fed a diet comprising 40% of the experimental final product in place of the fishmeal. The fish were fed these diets for 26 days. Waste products were collected and analyzed using an international accepted test protocol.

The results of these experiments showed high digestibility of feed, with 94.8% and 91.4% digested for the 20% and 40% inclusion rate feeds respectively, indicating a high degree of digestibility. These inclusion rates, 20% and 40%, are higher than normally would be included in a formulated diet and were formulated to test whether antinutritional properties are present and to see if there is a limit on digestibility. For palatability, the same groups of fish were studied for the time to initiate feeding and also the time to consume the total amount of food presented. Surprisingly, the diets comprising the experimental final product in lieu of fishmeal were consumed significantly faster than the control diets which comprised fishmeal. The results showed that the diets containing the 20% and 40% of the experimental final product were consumed in 66.2% and 35.0% of the time, respectively, relative to the time it took the fish to consume the control diet comprising fishmeal. Stated differently, this means fish will consume a diet which comprises 20% or 40% of the experimental final product in lieu of fishmeal 1.5 times and 2.85 times as fast, respectively, as a diet comprising fishmeal. This shows higher palatability for the experimental final product when compared to diets containing only fishmeal.

Because feed which is not consumed within a certain period of time is lost in industrial, artisanal, and hobbyist cultivation of aquatic species, the rate of feed consumption is an important metric. Because of this, attractants or substances which comprise improved attractant qualities are much sought after, as are those which encourage rapid consumption of feed due to taste, olfactory, or other characteristics. Likewise, with respect to the wild capture of fish, for sport or commercial purposes, baits with attractant qualities are highly desired, as well as baits which cause fish to strike.

In the foregoing specification, the invention is described with reference to specific embodiments thereof, but those skilled in the art will recognize that the invention is not limited thereto. Various features and aspects of the above-described invention may be used individually or jointly. Further, the invention can be utilized in any number of environments and applications beyond those described herein without departing from the broader spirit and scope of the specification. The specification and drawings are, accordingly, to be regarded as illustrative rather than restrictive. It will be recognized that the terms "comprising," "including," and "having," as used herein, are specifically intended to be read as open-ended terms of art.

## Claims

1. A method for producing a biomass, the method comprising:
providing a gas mixture to a consortium of bacteria and microbes in a bioreactor (100), the consortium including at least three of *Cupriavidus necator, Rhodobacter sphaeroides, Rhodopsuedamonas palustris, Rhodobacter capsulatus, Bacillus subtilis,* and *Bacillis magaterium,* the gas mixture including each of hydrogen gas, carbon dioxide gas, and oxygen gas, whereby hydrogen is metabolized by at least some of the bacteria of the consortium and growth of the consortium occurs solely by the fixation of carbon dioxide;
harvesting cells of the bacteria and microbes from the bioreactor (100); and
drying the solids to yield the biomass.

2. The method of claim 1, further comprising
separately receiving hydrogen gas and flue gas, the flue gas including both the carbon dioxide gas and the oxygen gas, and
mixing the hydrogen gas and flue gas to produce the gas mixture.

3. The method of claim 2, wherein, mixing the hydrogen gas and flue gas comprises diluting the flue gas approximately 5-fold with the hydrogen gas.

4. The method of claim 1, 2 or 3, wherein one of the hydrogen gas, carbon dioxide gas, and oxygen gas is introduced separately into the bioreactor (100) from the other two gases.

5. The method of any of the preceding claims, wherein the consortium of bacteria consists solely of *Cupriavidus necator, Rhodobacter sphaeroides, Rhodopsuedamonas palustris, Rhodobacter capsulatus,* and *Bacillus subtilis.*

6. The method of any of the preceding claims, wherein at least one of the bacterium of the consortium is of a strain that has been adapted over multiple generations to be tolerant of a flue gas, and wherein the gas mixture includes the flue gas.

7. The method of any of the preceding claims, further comprising, before providing the gas mixture to the consortium, adapting a bacterium of the consortium to a flue gas by growing multiple successive generations of the bacterium in the presence of the flue gas to produce an adapted bacterium, wherein the gas mixture includes the flue gas.

8. The method of any of the preceding claims, wherein one of the bacterium of the consortium expresses a carotenoid.

9. A product made by the method of any of the preceding claims.

10. A method for producing a feed product comprising: producing a biomass by
providing a gas mixture to a consortium of bacteria and microbes in a bioreactor (100), the consortium including at least three of *Cupriavidus necator, Rhodobacter sphaeroides, Rhodopsuedamonas palustris, Rhodobacter capsulatus, Bacillus subtilis,* and *Bacillis magaterium,* the gas mixture including each of hydrogen gas, carbon dioxide gas, and oxygen gas, whereby hydrogen is metabolized by at least some of the bacteria of the consortium and growth of the consortium occurs solely by the fixation of carbon dioxide,
harvesting cells of the bacteria and microbes from the bioreactor (100), dewatering, concentrating or drying the solids to yield the biomass; and blending the biomass with an additive.

11. A food or feed additive comprising:
a consortium including *Cupriavidus necator,*
a species from the genus *Rhodobacter,*
a species from the genus *Rhodopseudomonas,* and
a species from the genus *Bacillus.*

12. The food or feed additive of claim 11,
wherein the species from the genus *Rhodobacter* comprises *Rhodobacter sphearoidies,* and/or
wherein the species from the genus *Rhodopseudomonas* comprises *Rhodopseudomonas palustris.*

13. The food or feed additive of claim 11 or 12, wherein the species from the genus *Bacillus* comprises *bacillus subtilis.*

14. The food or feed additive of claim 12 or 13, further comprising *Rhodobacter capsulatus.*

15. The food or feed additive of any of the preceding claims 11 to 14, wherein the consortium is **characterized by** an enhanced attractiveness to fish and/or an enhanced palatability to fish.

16. The food or feed additive of any of the preceding claims 11 to 15, wherein the C. *necator* comprises over 85% of a dry weight of the consortium of the food or feed additive.
